# EUROPÄISCHE PATENTANMELDUNG

(11) **EP 0 742 220 A2**
(43) Veröffentlichungstag der Anmeldung: **13.11.1996**
(21) Anmeldenummer: 96106796.4
(22) Anmeldetag: 30.04.1996
(51) Int. Cl.: C07F 5/02, A61K 7/46

(54) **Borsäureester und ihre Verwendung als Riechstoffe**

(30) Priorität: 12.05.1995 DE 19517504
(71) Anmelder: HAARMANN & REIMER GMBH, D-37601 Holzminden (DE)
(72) Erfinder: Pelzer, Ralf, Dr., 37699 Fürstenberg (DE); Sturm, Wolfgang, Dr., Teterboro (US); Kochta, Joachim, Dr., 37603 Holzminden (DE)
(74) Vertreter: Petrovicki, Wolfgang, Dr.

(57) **Zusammenfassung**

Durch Luftfeuchtigkeit und/oder die von der Haut abgegebene Feuchtigkeit hydrolysierbare Ester von Riechstoffen, die eine alkoholische Hydroxylgruppe enthalten, zeigen nicht nur aufgrund des zeitlichen Ablaufs der Hydrolyse einen gegenüber den freien Alkoholen auftretenden "long lasting"-Effekt, sondern erzeugen darüber hinaus in Riechstoffzusammensetzungen einen viel klareren, saubereren Eindruck als die freien Alkohole.

## Beschreibung

Die Erfindung betrifft ein neues Prinzip für die Herstellung von Riechstoffen mit guter Haftung und lang anhaltender Wirkung.

Die Qualität von Parfumölen wird nicht nur durch die reinen Dufteigenschaften, die Duftintensität und die Stabilität im jeweiligen Anwendungsbereich, sondern auch durch das Haftvermögen des Parfumöls bestimmt, weil die Haftung für eine lang anhaltende Wirkung ausschlaggebend ist. Der Parfumeur bedient sich bei seinen kompositorischen Arbeiten seit langem sogenannter Fixateure, d.h. Verbindungen, die eine Verlängerung der Haftdauer von Parfumölen oder einzelner ihrer Bestandteile ermöglichen; vgl. z.B. W. Sturm und G. Mansfeld, Chemiker-Zeitung 99 (1975), 69. Die Fixateure können selbst Riechstoffe sein; sie können aber auch geruchlos sein.

Ein anderes Prinzip zur Herstellung von Riechstoffen mit lang anhaltender Wirkung beruht auf einer Mikroverkapselung der Riechstoffe, die erst durch Zerstörung der Kapselwand wieder freigesetzt werden; vgl. z.B. US-PS 3 971 852, EP-A 480 520.

Überraschenderweise wurde nun gefunden, daß durch Luftfeuchtigkeit und/oder die von der Haut abgegebene Feuchtigkeit hydrolysierbare Ester von Riechstoffen, die eine alkoholische Hydroxylgruppe enthalten, nicht nur aufgrund des zeitlichen Ablaufs der Hydrolyse einen gegenüber den freien Alkoholen auftretenden "long lasting"-Effekt zeigen, sondern darüber hinaus in Riechstoffzusammensetzungen einen viel klareren, saubereren Eindruck als die freien Alkohole erzeugen. Bevorzugte hydrolysierbare Ester sind die Borsäureester.

Gegenstand der Erfindung ist also ein Verfahren zur Haftungsverlängerung von Riechstoffen, die mindestens eine alkoholische Hydroxylgruppe besitzen, wonach man die Alkohole in Ester überführt, die bei mäßig rasch verlaufender Hydrolyse die alkoholischen Riechstoffe wieder freisetzen.

Gegenstand der Erfindung ist auch ein Verfahren zur Herstellung von Parfumölen mit verlängerter Haftung unter Mitverwendung von Borsäureestern von Verbindungen, die mindestens eine alkoholische Hydroxylgruppe besitzen.

Weiterer Gegenstand der Erfindung ist die Verwendung von Borsäureestern von Verbindungen, die mindestens eine alkoholische Hydroxylgruppe besitzen, als Riechstoffe.

Weiterer Gegenstand der Erfindung ist die Verwendung von Borsäureestern von Riechstoffen, die mindestens eine alkoholische Hydroxylgruppe enthalten, zur Herstellung desodorierender Mittel.

Weiterer Gegenstand der Erfindung sind schließlich neue Borsäureester.

Bevorzugte Borsäureester entsprechen der Formel

B(OR¹)(OR²)(OR³) (I)

worin
- R¹ bis R³: unabhängig voneinander für Wasserstoff oder für offenkettige, cyclische oder bicyclische gesättigte oder einfach oder mehrfach olefinisch ungesättigte oder aromatische Kohlenwasserstoffreste stehen, die ein- bis dreimal durch C₁-C₄-Alkoxy oder Amino substituiert sein können,
mit der Maßgabe, daß mindestens einer der 3 Reste mindestens 5 C-Atome enthält.

Bevorzugte Reste R¹ bis R³ umfassen also Alkyl, Cycloalkyl und Aralkyl mit 5 bis 15 C-Atomen, wobei die Reste, die keinen aromatischen Ring enthalten, besonders bevorzugt sind.

Die bevorzugten Borsäureester zeigen eine gute Stabilität in Formulierungen bei gleichzeitig hinreichend gleichmäßiger Hydrolyse an der Luft und mithin hohem Retentionsvermögen der gebundenen Alkohole. Ein vergleichbarer Retentionseffekt ist bisher nur durch den Einsatz mikroverkapselter Riechstoffe zu erzielen gewesen, was jedoch neben gelegentlichen Formulierungsschwierigkeiten auch zur Verwendung von erheblichen Mengen an Verkapselungsmatrices zwingt.

Für die Herstellung der Borsäureester bevorzugte Alkohole umfassen die folgenden Verbindungen:

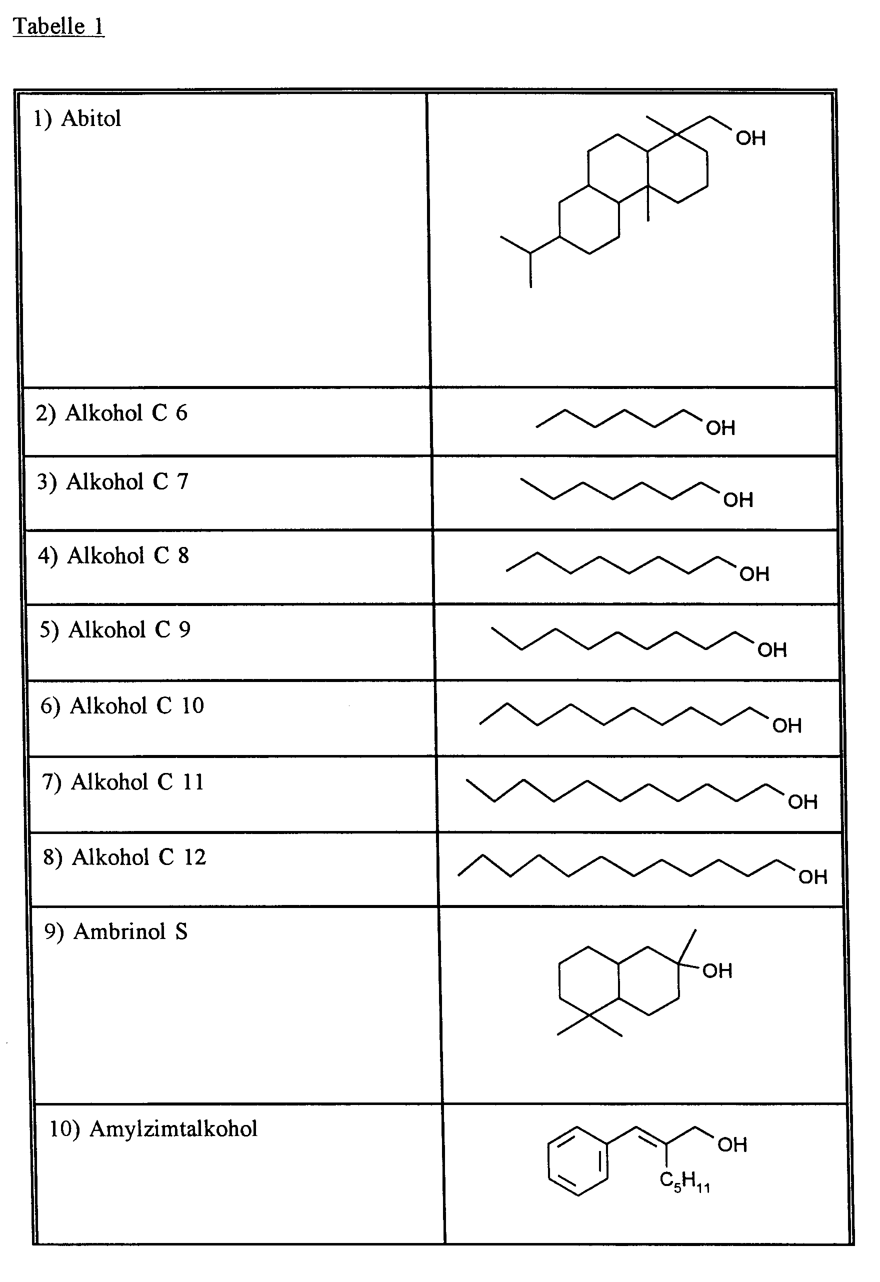

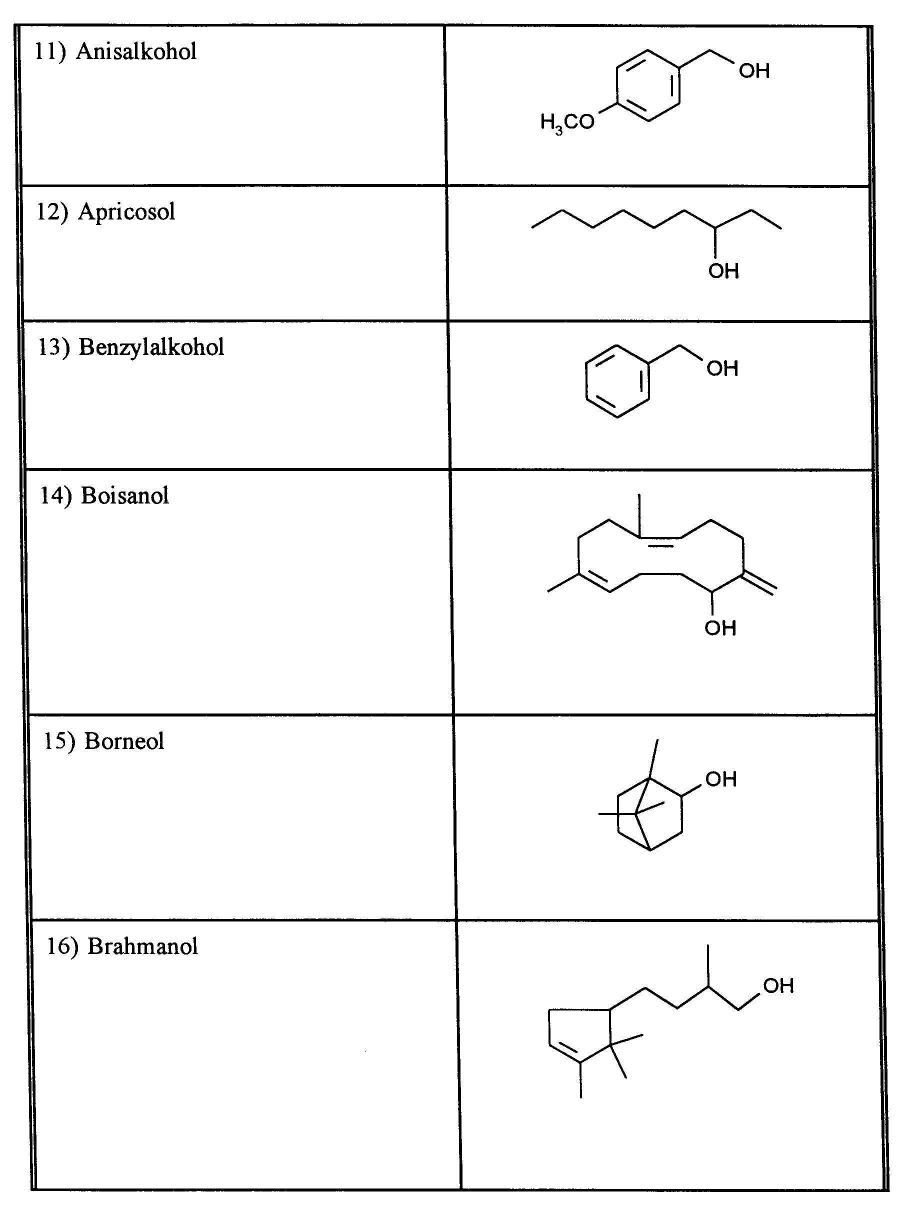

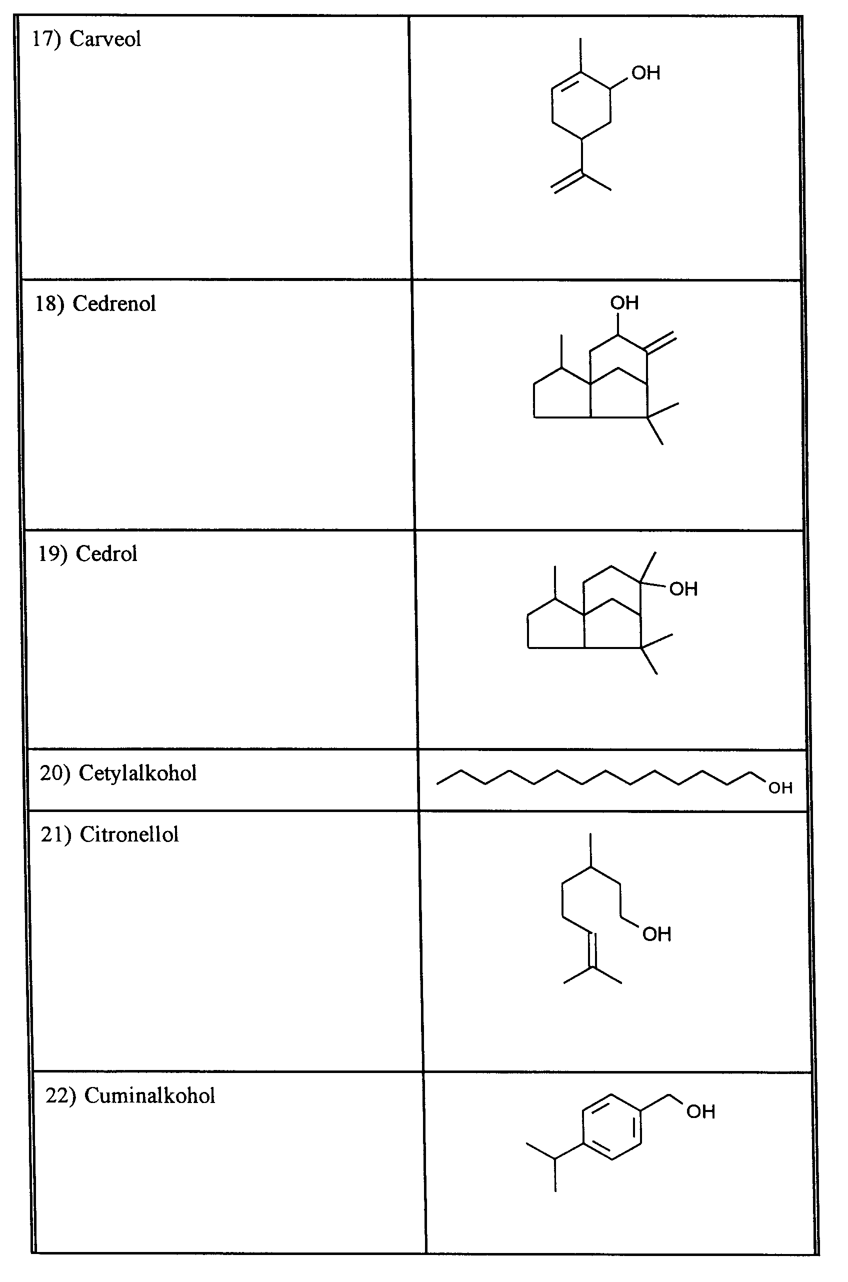

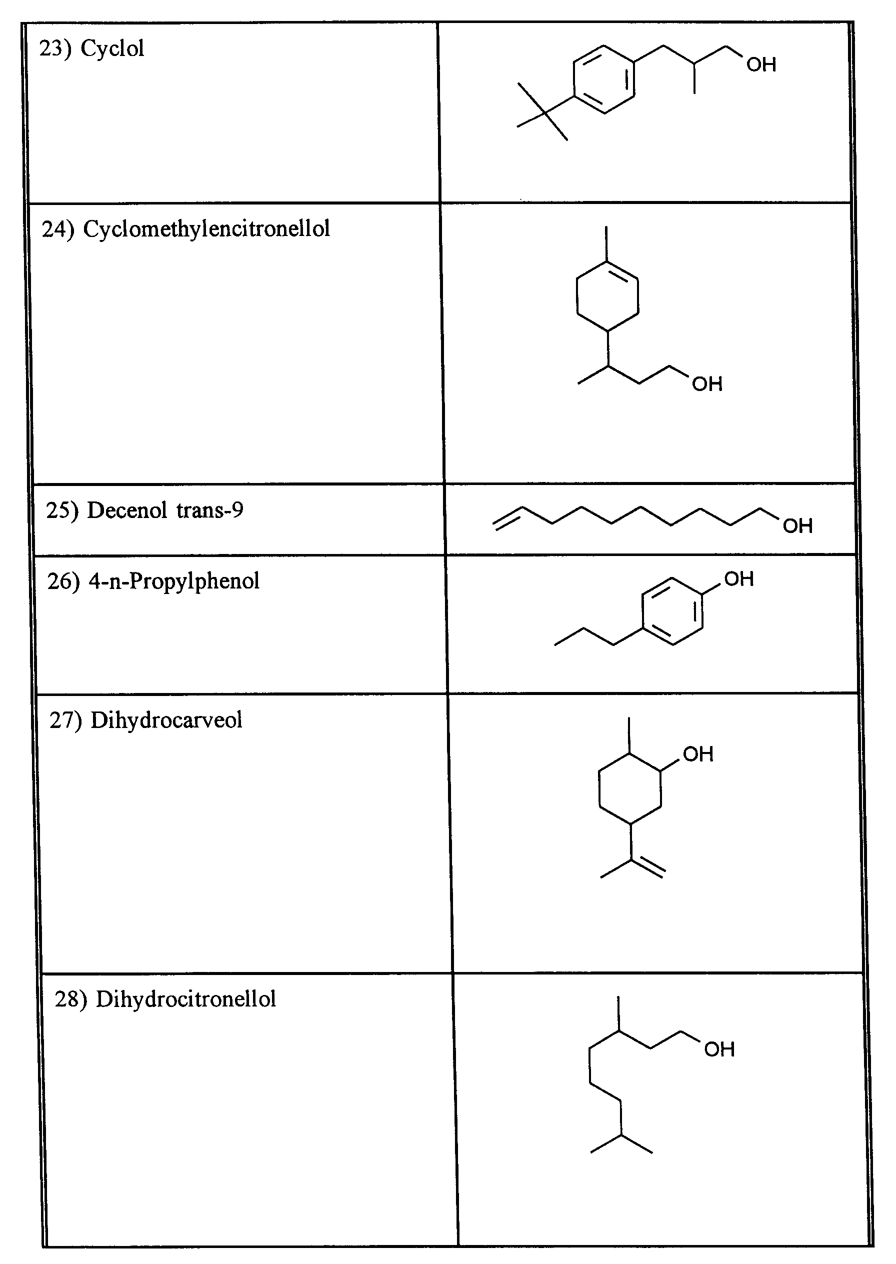

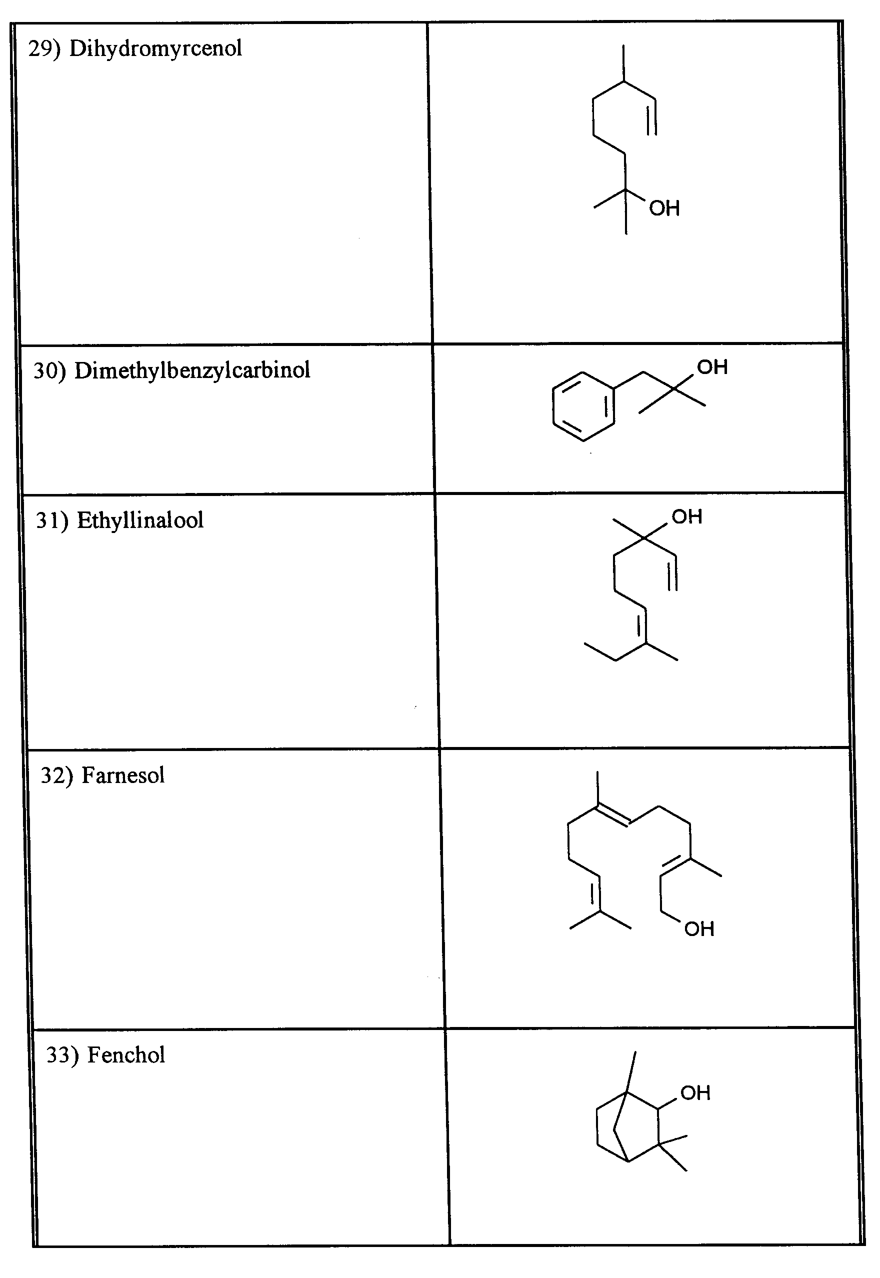

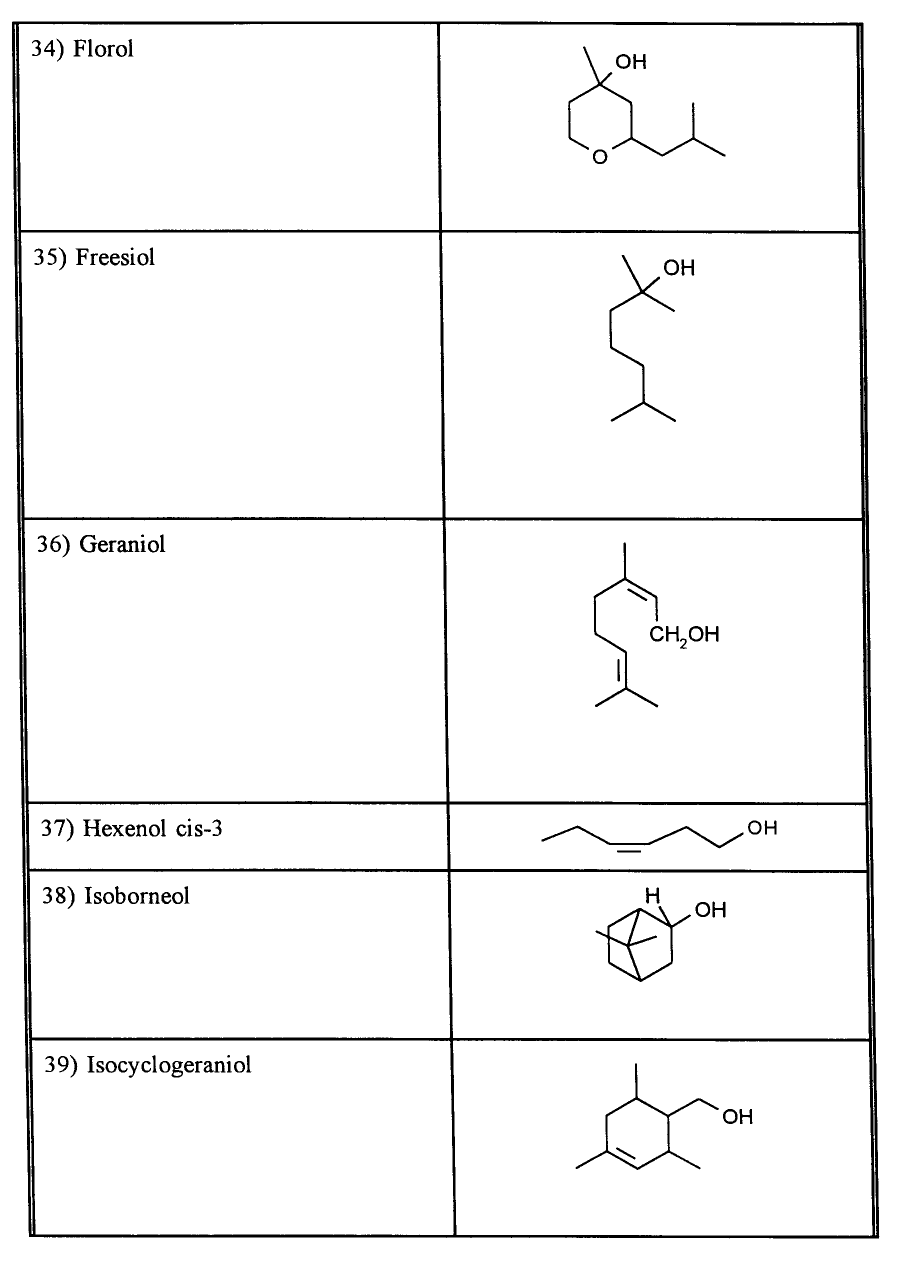

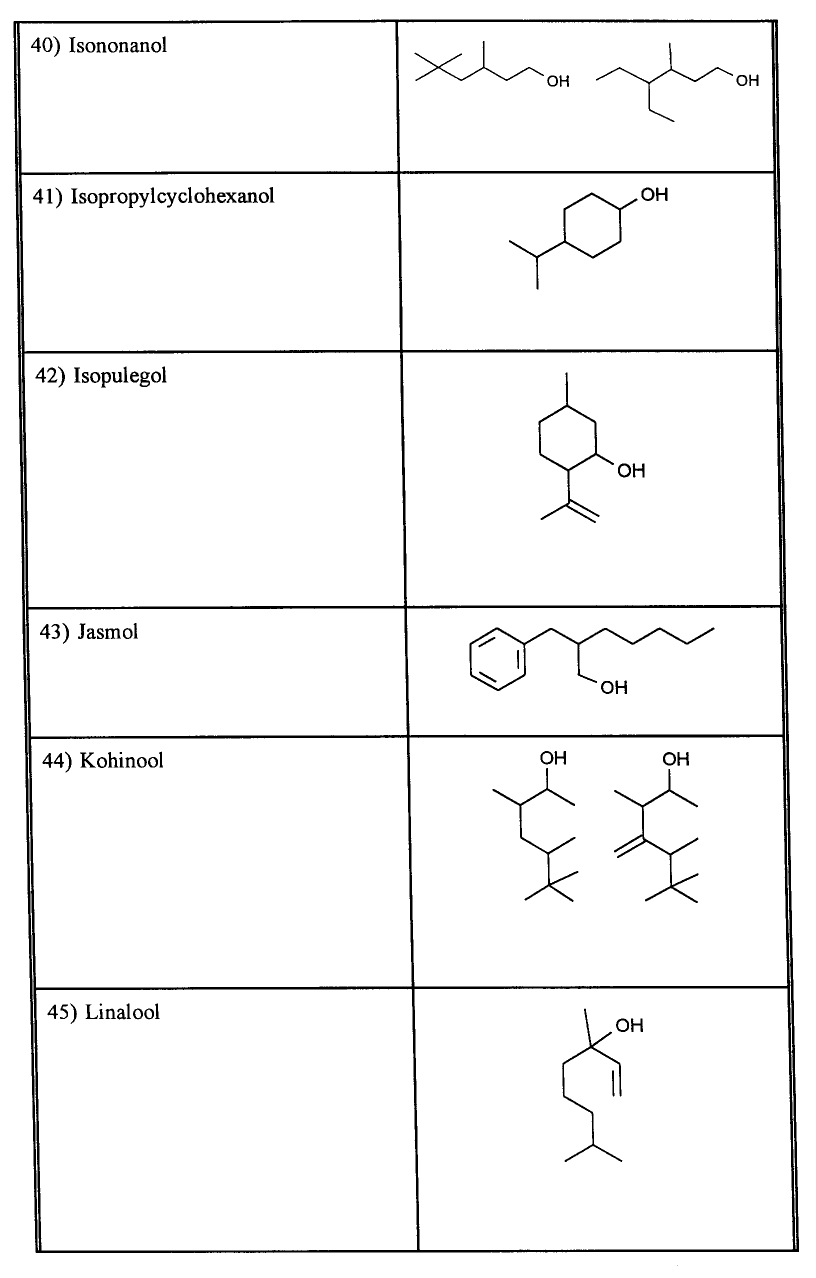

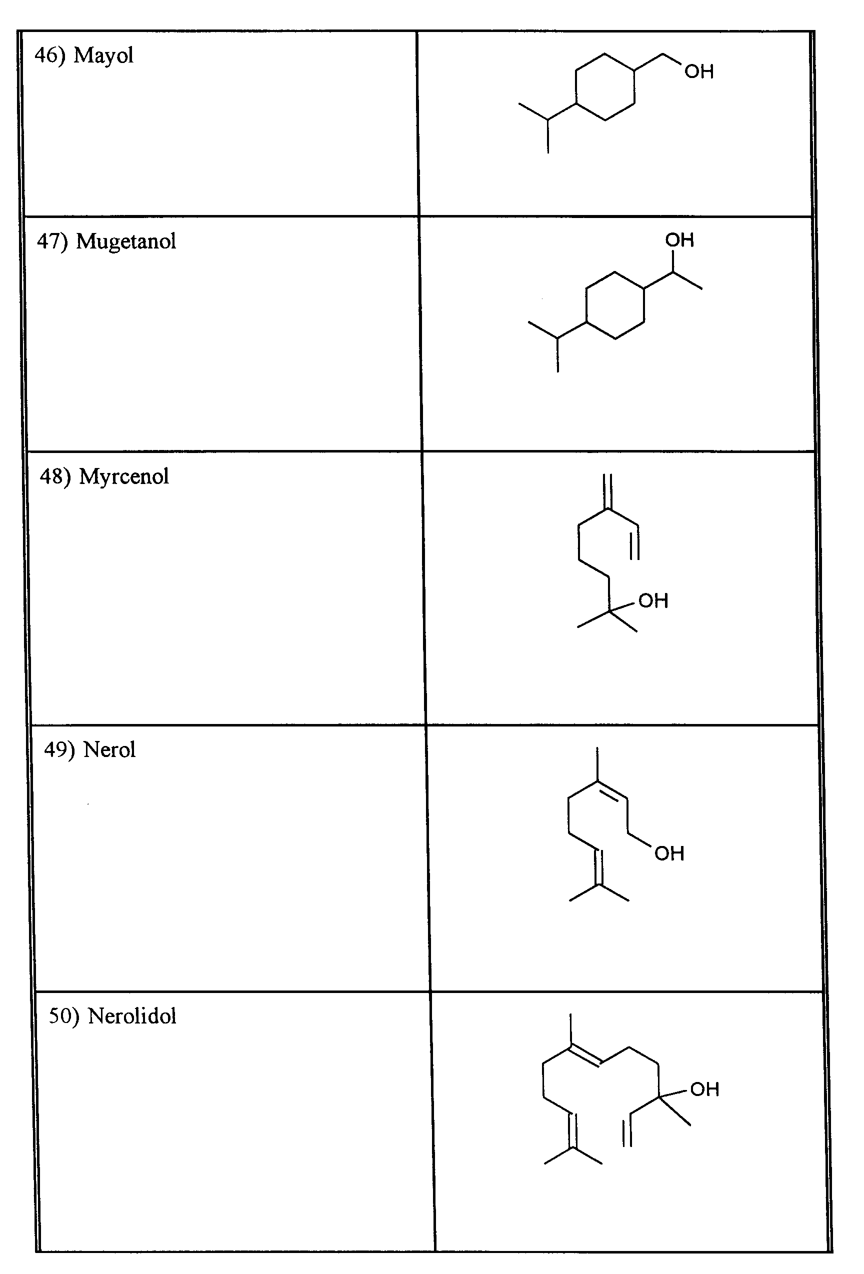

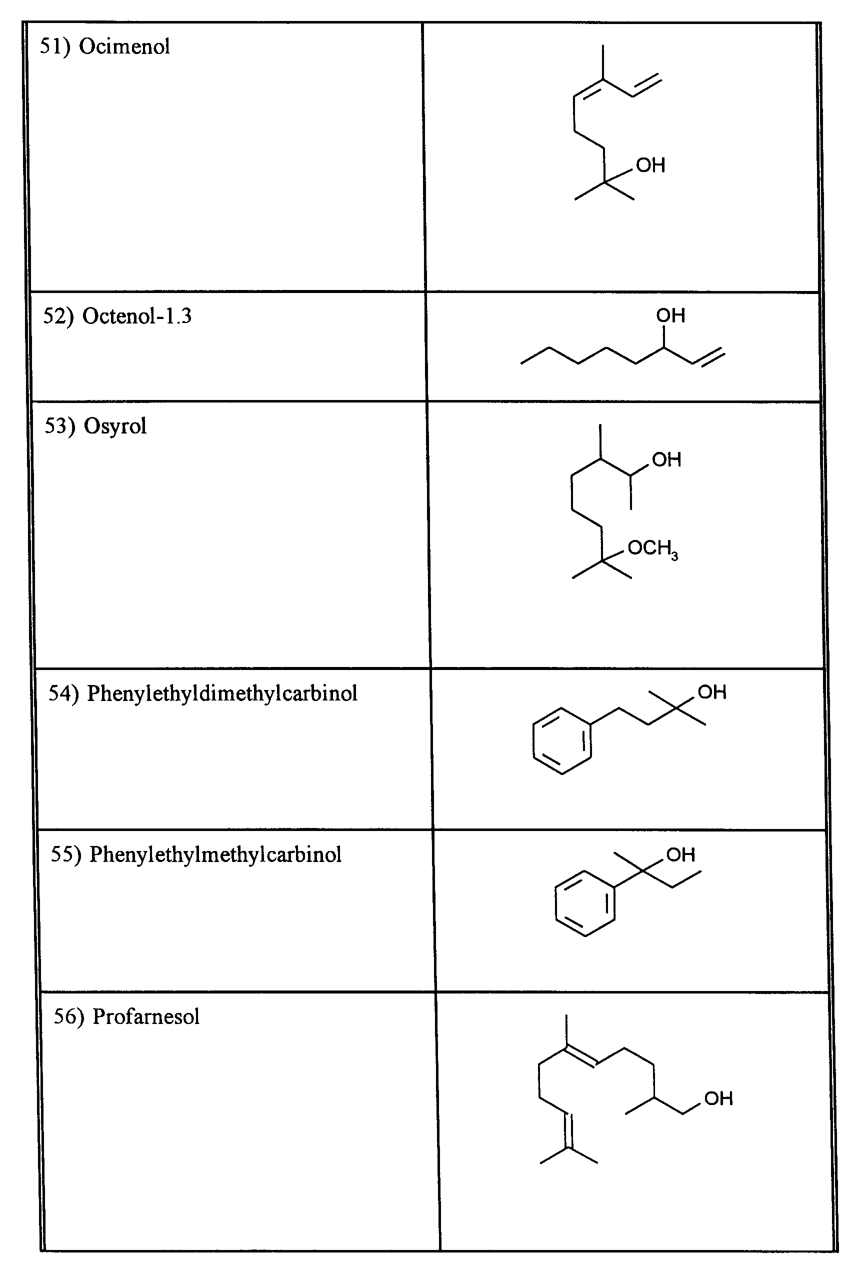

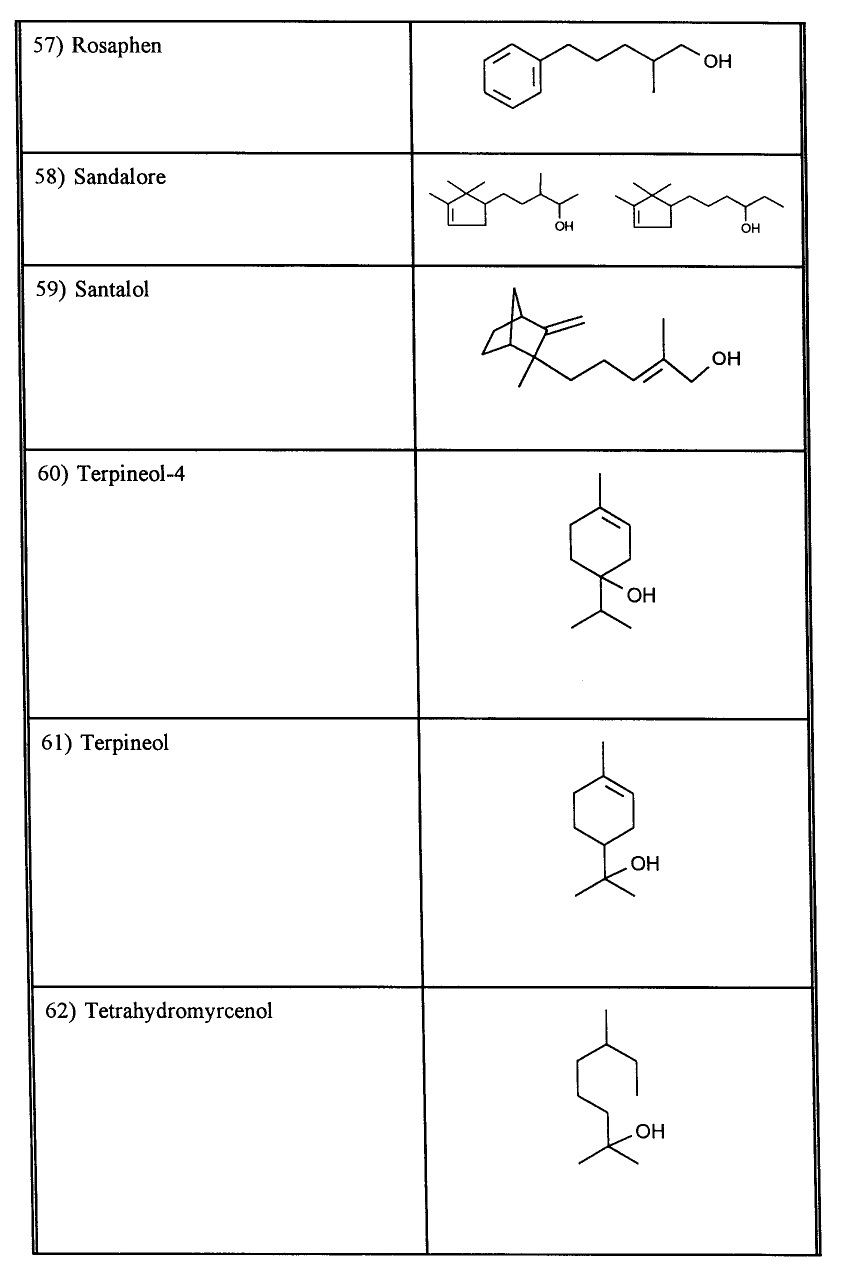

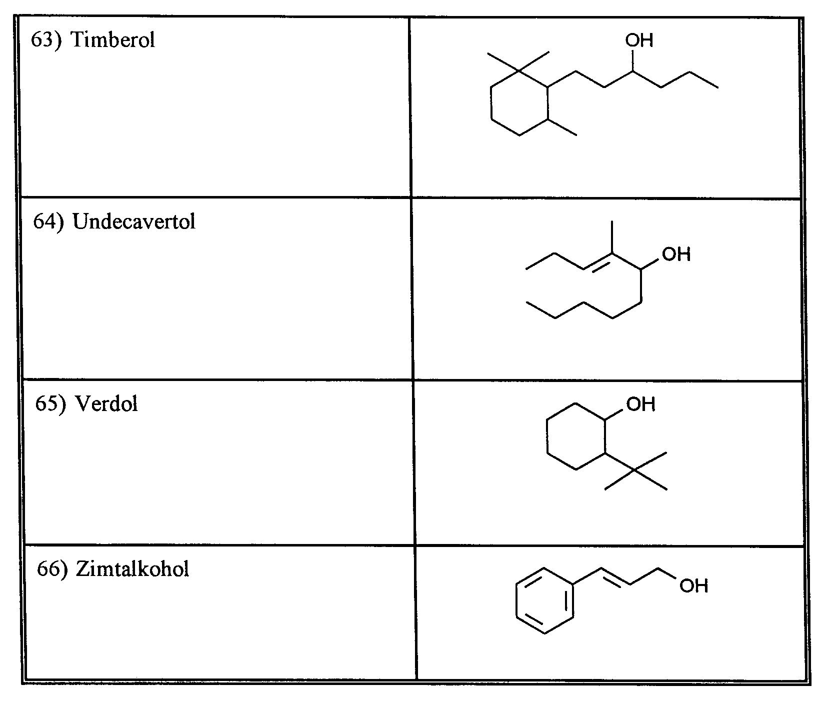

Die Borsäureester können einheitlich sein, d.h. auf einem einzigen Alkohol basieren, oder aus zwei oder drei verschiedenen Alkoholen hergestellt sein. Die Borsäureester können auch ein oder zwei freie Hydroxylgruppen am Boratom enthalten.

Die Herstellung der Borsäureester kann auf übliche Weise durch Veresterung von Borsäure oder Bortrioxid erfolgen.

Die Borsäureester können einzeln oder in Form Borsäureester enthaltender Parfumkompositionen eingesetzt werden, und zwar sowohl in Parfums als auch in Parfum enthaltenden, vorzugsweise kosmetischen Formulierungen. Als solche kommen beispielsweise in Betracht:
Tagescremes (meistens Öl-in-Wasser-Emulsionen),
Nachtcremes (meistens Wasser-in-Öl-Emulsionen),
Sonnenschutzcremes,
Duschgele,
Shampoos,
Haargele,
Enthaarungscremes,
Körperpuder,
Fußpflegemittel und
Körpermilch.

Besonders von Vorteil sind die erfindungsgemäß zu verwendenden Borsäureester in desodorierenden Mitteln, bei denen nach bestimmungsgemäßem Gebrauch, z.B. in der Achselhöhle, durch den auftretenden Schweiß die zur Hydrolyse notwendige Feuchtigkeit erzeugt wird und die Duftfreisetzung erst dann erfolgt, wenn sie auch benötigt wird.

Die desodorierenden Mittel können daneben üblicherweise eingesetzte Additive, wie z.B. Aluminiumsalze bzw. Aluminium-Zirkoniumverbindungen (in diesem Fall handelt es sich um sogenannte Antitranspirant-/Antiperspirantformulierungen) aber auch bakteriostatisch wirkende Substanzen, wie z.B. 2,4,4'-Trichlor-2'-hydroxydiphenylether oder Trichlorcarbanilid enthalten.

Die folgenden Beispiele zeigen die Herstellung desodorierender Mittel. Die Rezepturen enthalten Bezeichnungen nach CTFA-Nomenklatur. Prozentangaben beziehen sich jeweils auf das Gewicht.

### Beispiele

### Darstellung der Borsäureester

10 Mol des Alkohols werden im Reaktionsgefäß vorgelegt und mit 1 l Toluol versetzt. Zu diesem Gemisch gibt man 3,4 Mol Borsäure und kreist am Wasserabscheider aus, bis mindestens 9,5 Mol Wasser gebildet wurden. Anschließend wird das Toluol abdestilliert und der Reaktionsrückstand über einen Dünnschichtverdampfer vom Alkohol befreit. Der so zugängliche Borsäureester ist von hinreichender Reinheit, so daß weitere Aufarbeitungsschritte nicht notwendig sind.

Als Alkoholkomponente sind alle Riechstoffe mit OH-Funktionalität geeignet, Beispiele bietet hier Tabelle 1.

Zum Schleppen des Wassers sind Lösungsmittel zu verwenden, die hinreichende Azeotrope mit Wasser bilden. Dies können Toluol, Xylol, Dekalin u.v.a. Lösungsmittel sein, es können aber auch die Alkohole im Überschuß eingesetzt werden, wenn sie hinreichende Azeotrope bilden. Der bevorzugte Siedebereich sollte zwischen 50°C und 250°C liegen.

### Beispiel 1: Antiperspirant-Spray ohne Alkohol

| | [%] |
|---|---|
| Aluminium-Chlorhydrat | 3,0 |
| Kieselgel | 0,2 |
| Cyclomethicone | 3,0 |
| Capryl-/Caprinsäure-triglycerid | 3,0 |
| tris-cis-3-Hexenylborat | 0,8 |
| Butan, 1,4 bar | 90,0 |

### Beispiel 2: Deo roll-on

| | | [%] |
|---|---|---|
| Phase A: | Kieselgel Aerosil 200 V | 1,0 |
| | Mono-/Diglycerid der Laurinsäure | 3,0 |
| | Dimethicone (Polydimethylsiloxan) | 5,0 |
| Phase B: | Baysilon Oil COM 20000 | 90,0 |
| Phase C: | Tris-(phenylethyl)-borat | 1,0 |

Phasen A auf 70 bis 75°C erhitzen, unter Rühren B zu A geben, bei ca. 35°C Phase C zugeben, unter Rühren erkalten lassen.

### Beispiel 3: Deodorantspray ohne Kieselgel

| | [%] |
|---|---|
| Aluminium-Chlorhydrat | 3,3 |
| Cyclomethicone | 3,0 |
| Capryl-/Caprinsäure-triglycerid | 3,0 |
| tris-Linalylborat | 1,0 |
| Butan | 89,7 |

### Beispiel 4: Deodorant-Stift, transparent, ohne Alkohol

| | [%] |
|---|---|
| Natriumstearat | 8,0 |
| Propylenglykol | 29,4 |
| Dipropylenglykol | 10,0 |
| Glycerin | 49,0 |
| Oleylalkohol-Polyethylenglykol-ether | 2,0 |
| 2,4,4'-Trichlor-2'-hydroxy-diphenylether | 0,1 |
| tris-Geranylborat | 1,0 |

### Beispiel 5: Deodorant-Trockenstift, Suspensionstyp

| | [%] |
|---|---|
| Cyclomethicone | 49,0 |
| Aluminium-Chlorhydrat | 10,0 |
| Talkum | 15,3 |
| Stearylalkohol | 18,5 |
| Polyethylenglykol (durchschnittlich 20 Ethylenoxidgruppen) | 4,5 |
| Glycerylstearat | 0,5 |
| Polyethylenglykolstearat (durchschnittlich 20 Ethylenoxidgruppen) | 1,0 |
| Triclosan | 0,2 |
| tris-Hydroxycitronellalborat | 1,0 |

### Beispiel 6: (Vergleich) Antiperspirant-Spray ohne Alkohol

| | | [%] |
|---|---|---|
| A | Aluminium-Chlorhydrat | 3,0 |
| | Kieselgel | 0,2 |
| | Cyclomethicone | 3,0 |
| | Capryl-/Caprinsäure-triglycerid | 3,0 |
| | cis-3-Hexenol | 0,8 |
| B | Butan, 1,4 bar | 90,0 |

Die verzögerte Freisetzung von cis-3-Hexenol aus der Formulierung nach dem erfindungsgemäßen Beispiel 1), bei der das Borat zur Anwendung kam, konnte im Vergleich zur obigen Formulierung, die freies Cis-3-Hexenol enthielt, durch dynamische Headspace-Gaschromatographie deutlich dokumentiert werden.

Bei der genannten HS-GC-Untersuchungsmethode wurde nach Einbringung der entsprechenden Formulierung auf Transpore-tape® (Firma 3 M) und nachfolgender HS-GC-Messung der Gehalt an freiem cis-3-Hexenol im Dampfraum in Abhängigkeit von der Zeit aufgezeichnet. Es wurde gefunden, daß im Falle der Verwendung des freien Alkohols bereits nach 4 Stunden keine Substanz mehr im Dampfraum nachweisbar ist.

Dieser Befund deckt sich mit entsprechend olfaktorisch ermittelten Werten.

Im Gegensatz hierzu beobachtet man im Falle des eingesetzten Borates auch nach 4 Stunden sowohl analytisch mit dem oben beschriebenen HS-GC-Gerät als auch geruchlich einen deutlichen Gehalt an cis-3-Hexenol.

Dieser analytische Befund konnte ebenfalls durch olfaktorische Evaluation nach Applikation der Formulierungen nach Beispiel 1) und 6) unter die Achsel bestätigt werden.

Hierbei wurde folgende Procedere angewendet:

Ein nach Beispiel 1) hergestelltes Deodorant mit tris-(cis)-3-Hexenylborat wurde bei 3 Personen (2 männlich, 1 weiblich) unter die eine, ein nach Beispiel 6) hergestelltes Deodorant mit freiem cis-3-Hexenol unter die anderen Achsel appliziert. Dabei wurde keine Festlegung nach linker bzw. rechter Achsel für eines der beiden Produkte vorgenommen (sog. Doppelblindtest).

Anschließend wurde von insgesamt 10 Personen jede Stunde abgerochen und das Geruchsergebnis hinsichtlich der Geruchsintensität notiert.

Die Resultate wurden abschließend gemittelt.

Dieser Praxistest wurde mit insgesamt 5 Boraten bzw. deren freien Alkoholen, eingearbeitet in Formulierungen analog Beispiel 1) und 6), durchgeführt. Die Ergebnisse sind in Tabelle 2 dargelegt.

**Tabelle 2**

| Geruchsergebnis (Impact) | | | |
|---|---|---|---|
| | freier Alkohol | Borat | nach Stunden |
| cis-Hexenol | 0,0 | 3,2 | 0,5 |
| Linalool | 0,2 | 2,8 | 2 |
| Geraniol | 0,7 | 1,9 | 2 |
| Phenylethylalkohol | 1,5 | 3,9 | 2 |
| Hydroxycitronellal | 0,4 | 1,8 | 2 |

- Bewertung:: 5 = sehr starker Geruch
4 = starker Geruch
3 = mittlerer Geruch
2 = schwacher Geruch
1 = sehr schwacher Geruch
0 = kein Geruch

### Beispiel 7

Über den Effekt des hohen Retentionsverhaltens hinaus zeigen die Borate eine sehr gute antimikrobielle Wirksamkeit, was sie insbesondere für den Einsatz in Deo- oder Körperpflegeprodukten geeignet macht.

Die antimikrobiellen Eigenschaften konnten beispielhaft an folgenden erfindungsgemäßen Boraten aufgezeigt werden:
- tris-(cis)-3-Hexenylborat
- tris-Geranylborat
- tris-Linalylborat
- tris-Nerylborat
- tris-Phenylethylborat
- tris-Undecenylborat
- tris-Hydroxycitronellalborat

Sowohl gegenüber gramnegativen Bakterien (z.B. Pseudomonas aeruginosa) als auch grampositiven Bakterien (z.B. Staphylococcus aureus) zeigen die beispielhaft genannten Borate eine hervorragende wachstumshemmende Wirkung, was experimentell durch einen sogenannten Mikrotiterplattentest auf sterilem Agar (Inkubationszeit 3 Tage, Temperatur 37°C) dokumentiert werden konnte.

Neben den mikrobiologischen Eigenschaften zeigen die Borsäureester aufgrund ihres chemischen Aufbaus eine ausgezeichnete Löslichkeit in Parfumkompositionen und demnach auch lipophilen Formulierungen.

### Beispiele 8 und 9

Aufgrund ihres hohen Siedepunktes bewirken die erfindungsgemäßen Borate neben der gewünschten Retention des verwendeten Alkohols auch eine stärkere Fixierung und demzufolge längere Duftwirkung der übrigen Riechstoffe der Parfumölkomposition. Dies läßt sich gut in der Gegenüberstellung der Rezepturen aus den Beispielen 8 und 9 zeigen.

### Beispiel 8: Parfumöl mit freien Alkoholen

| | [%] | |
|---|---|---|
| Hexenol cis-3 | 0,1 | |
| Dihydromyrcenol | 5,5 | |
| Lavandinöl | 4,5 | |
| Nerolidol | 6,0 | |
| Farnesol | 7,0 | |
| Linalool | 12,0 | |
| Phenylethylalkohol | 4,5 | |
| Geraniol | 2,0 | |
| Citronellol | 4,0 | |
| Nerol Supra | 1,0 | |
| Benzylacetat | 2,2 | |
| Cumarin | 4,5 | |
| Cedrol krist. | 0,5 | |
| Chromanolid 50 % in BB | 40,0 | BB = Benzylbenzoat |
| Jasmol | 6,2 | |

### Beispiel 9

| | [%] |
|---|---|
| tris-cis-Hexenylborat | 0,1 |
| Dihydromyrcenol | 5,5 |
| Lavandinöl | 4,5 |
| Nerolidol | 6,0 |
| Farnesol | 7,0 |
| tris-Linalylborat | 12,0 |
| tris-Phenylethylborat | 4,5 |
| tris-Geranylborat | 2,0 |
| Citronellol | 4,0 |
| Nerol Supra | 1,0 |
| Benzylacetat | 2,2 |
| Cumarin | 4,5 |
| Cedrol krist. | 0,5 |
| Chromanolid 50 % in BB | 40,0 |
| Jasmol | 6,2 |

Durch den Einsatz der erfindungsgemäßen Borate wird eine deutliche Retention der Kopfnotenkomponenten und eine signifikant längere Duftwirkung erreicht, was olfaktorisch gezeigt werden konnte.

## Patentansprüche

1. Verfahren zur Haftungsverlängerung von Riechstoffen, die mindestens eine alkoholische Hydroxylgruppe besitzen, wonach man die Alkohole in Ester überführt, die bei mäßig rasch verlaufender Hydrolyse die alkoholischen Riechstoffe wieder freisetzen.

2. Verfahren zur Herstellung von Parfumölen mit verlängerter Haftung unter Mitverwendung von Borsäureestern von Verbindungen, die mindestens eine alkoholische Hydroxylgruppe besitzen.

3. Verwendung von Borsäureestern von Verbindungen, die mindestens eine alkoholische Hydroxylgruppe besitzen, als Riechstoffe.

4. Verwendung von Borsäureestern von Riechstoffen, die mindestens eine alkoholische Hydroxylgruppe enthalten, zur Herstellung desodorierender Mittel.

5. Borsäureester entsprechend der Formel
B(OR¹)(OR²)(OR³) (I)
worin
R¹ bis R³ unabhängig voneinander für Wasserstoff oder für offenkettige, cyclische oder bicyclische gesättigte oder einfach oder mehrfach olefinisch ungesättigte oder aromatische Kohlenwasserstoffreste stehen, die ein- bis dreimal durch C₁-C₄-Alkoxy oder Amino substituiert sein können,
mit der Maßgabe, daß mindestens einer der 3 Reste mindestens 5 C-Atome enthält.

6. Borsäureester nach Anspruch 5 aus Alkoholen R¹OH, R²OH, R³OH, wobei diese Alkohole aus den Verbindungen 9 bis 12, 17 bis 19, 22 bis 29, 31 bis 35, 37, 39 bis 41, 43 bis 51 und 53 bis 64 in Tabelle 1 der Beschreibung ausgewählt sind.
